# EUROPEAN PATENT SPECIFICATION

(11) **EP 2 623 102 B1**
(45) Date of publication and mention of the grant of the patent: **09.01.2019**
(21) Application number: 11829254.9
(22) Date of filing: 29.09.2011
(51) Int. Cl.: A61K 31/445, A61K 9/06, A61K 9/08, A61K 9/70, A61K 47/10, A61K 47/12, A61K 47/14

(54) **TRANSDERMAL ABSORPTION PREPARATION**
PRÄPARAT ZUR TRANSDERMALEN ABSORPTION
PRÉPARATION POUR ABSORPTION TRANSDERMIQUE

(30) Priority: 23.03.2011 JP 2011064431; 30.09.2010 JP 2010221604
(43) Date of publication of application: 07.08.2013
(73) Proprietor: FUJIFILM Toyama Chemical Co., Ltd., Tokyo 104-0031 (JP)
(72) Inventor: KUBO, Yoshiko, Toyama-shi Toyama 930-8508 (JP); KOUNO, Ryoji, Toyama-shi Toyama 930-8508 (JP); YOSHIMURA, Misaki, Toyama-shi Toyama 930-8508 (JP); JO, Rumi, Toyama-shi Toyama 930-8508 (JP)
(74) Representative: Hartz, Nikolai
(86) International application number: PCT/JP2011/072334
(87) International publication number: WO 2012/043701

(56) References cited:
- EP-A1- 1 767 526
- WO-A1-93/08798
- WO-A1-03/074476
- WO-A1-2006/003881
- WO-A1-2006/011499
- WO-A2-2006/021833
- US-A1- 2008 153 885
- MITSUYAMA JUNICHI ET AL: "In vitro and in vivo antifungal activities of T-2307, a novel arylamidine", ANTIMICROBIAL AGENTS AND CHEMOTHERAPY, AMERICAN SOCIETY FOR MICROBIOLOGY, vol. 52, no. 4, 1 April 2008 (2008-04-01), pages 1318-1324, XP002557123, ISSN: 0066-4804, DOI: 10.1128/AAC.01159-07
- MURANISHI S: "Absorption enhancers", CRITICAL REVIEWS IN THERAPEUTIC DRUG CARRIER SYS, BEGELL HOUSE PUBLISHING INC, US, vol. 7, no. 1, 1 January 1990 (1990-01-01) , pages 1-33, XP008117820, ISSN: 0743-4863

## Description

### Technical Field

The present invention relates to a transdermal preparation of an arylamidine derivative useful for treating fungal infections.

### Background Art

Serious deep mycosis such as invasive candidiasis can often be a fatal disease. In the past, it has been considered that the principal protective mechanism on the side of a host organism against fungi such as Candida is nonspecific immunization by neutrophils. When this protective mechanism functions normally there is little risk of becoming infected with fungi. However, in recent years, the risk of suffering from deep mycosis has been boosted because of the increased number of patients with underlying diseases decreasing the immunological function of the body, such as malignant tumors (in particular, hemopoietic malignant tumors such as acute leukemia or malignant lymphoma) and AIDS, frequent use of anticancer agents or immunosuppressants, heavy use of antibacterial antibiotics or steroid hormones, long-term use of central venous hyperalimentation or venous catheterization and the like (Non-Patent Document 1).

Agents used for the treatment of such deep mycosis are very few, when compared to antibacterial agents used, and include only amphotericin B, flucytosine, miconazole, fluconazole, fosfluconazole, itraconazole, voriconazole, micafungin and the like.

On the other hand, there is an increasing need for safe and effective agents against opportunistic fungal infections caused by fungal pathogens such as Candida, Cryptococcus and Aspergillus.

For example, amphotericin B, has an extremely strong fungicidal action, it has a problem regarding side effects such as nephrotoxicity, so that its clinical usage is limited. Flucytosine has problems with the development of resistance. Micafungin has a low activity against the Cryptococcus. Azoles such as fluconazole and voriconazole are most frequently used at present due to their balance between effectiveness and safety, although their fungicidal action is inferior to that of amphotericin B (Non-Patent Documents 2 and 3).

Transdermal preparations have the following advantages (Non-Patent Documents 4, 5). (1) Sustained drug delivery at a controlled rate is available. (2) The first pass effect in the liver can be avoided. (3) No pain is associated with administration.

The stratum corneum of the skin surface serves as a barrier for preventing various chemical substances externally present from invading into a body (Non-Patent Document 6). Because of the function, many drugs have low skin permeability. Thus, it is difficult to absorb a sufficient amount of drug to exert a pharmacological effect into a body simply by applying the drug to the skin surface (Non-Patent Document 7). Further, since the stratum corneum is a highly lipophilic membrane, there is a tendency that a compound highly soluble in lipid (octanol/water partition coefficient is 100 to 1000) is easier to permeate through the membrane whereas a compound highly soluble in water is more difficult to permeate through the membrane (Non-Patent Documents 6, 8). It is an important problem in developing a transdermal preparation how the strong barrier function of the stratum corneum is overcome (Non-Patent Document 9). To overcome such difficulties, many methods for enhancing transdermal absorption have been studied and reported. However, the effect of the transdermal absorption enhancing significantly varies depending upon the drug and it is extremely difficult to find out a method for enhancing transdermal absorption appropriate for a novel compound from data in the past (Non-Patent Document 10).

4-{3-[4-(3-{4-[Amino(imino)methyl]phenoxylpropyl)-1-piperidinyl]propoxy}benzamidine (hereinafter referred to as "compound A") or a salt thereof has a strong antifungal activity and also has an effect upon an azole antifungal agent-resistant fungi (Patent Document 1). However, the octanol/water partition coefficient of compound A in the range of pH 3 to 9 is less than 0.05, which greatly differs from the partition coefficient (100 to 1000) of a compound generally having high membrane permeability. Compound A tends to be difficult to permeate through a membrane. Thus, it is difficult to provide a transdermal preparation comprising compound A or a salt thereof and having transdermal absorbability sufficient to exert a pharmacological effect. A transdermal preparation of compound A or a salt thereof is not known.

### Prior Art Documents

### Patent Document

Patent Document 1: International Publication No. WO 2006/003881 pamphlet

### Non-Patent Documents

Non Patent Document 1: Rinsho to Biseibutsu (Clinical Microbiology), 1990, Vol. 17, pages 265-266
Non Patent Document 2: Rinsho to Biseibutsu (Clinical Microbiology), 1994, Vol. 21, Pages 277-283
Non Patent Document 3: Rinsho to Biseibutsu (Clinical Microbiology), 2003, Vol. 30, Pages 595-614
Non Patent Document 4: "Latest Transdermal Agent -from fundamental of development to point of application-", Information mechanism, 2008, page 41
Non Patent Document 5: "Development of Medicine, Vol. 13, Drug Delivery Method", Hirokawa Shoten, Co. Ltd., 1989, page 87
Non Patent Document 6: "Latest Transdermal Agent -from fundamental of development to point of application-", Information mechanism, 2008, Pages 7-8, page 43
Non Patent Document 7: "Today's DDS -Drug Delivery System-", Iyaku Journal Co., Ltd., 1999, page 214
Non Patent Document 8: Journal of JSPME (Journal Society of Pharmaceutical Machinery and Engineering), 2011, Vol. 20, page 71
Non Patent Document 9: "Latest Dosage Form and Preparation Design Know-How of Pharmaceutical Preparations", Technical Information Institute, 2005, page 343
Non Patent Document 10: "Today's DDS -Drug Delivery System-", Iyaku Journal Co., Ltd., 1999, page 218

### Summary of Invention

### Problem to be Solved by the Invention

The development of a transdermal preparation useful for treating fungal infections for improving quality of life of patients has been desired.

### Means for Solving Problem

Under such circumstances, as a result of intensive study, the present inventors discovered that a preparation comprising compound A or a salt thereof as an active ingredient and a transdermal absorption enhancer as defined in the claims has excellent skin permeability, thereby arriving at the present invention.

### Advantageous Effects of the Invention

The preparation of the present invention has one or more of the following advantages. (1) Owing to excellent skin permeability, the pharmacological effect of compound A can be exerted by transdermal administration. (2)The blood concentration of compound A can be maintained at an appropriate level for a long time. (3) If an unexpected side effect is developed, administration can be easily stopped. (4) Compliance can be improved. (5) Owing to simple administration method, the reduction of burden of medical staff in therapy can be expected. (6) Since administration in home treatment is available, the quality of life of patients can be significantly improved. (7) Temporal stability is excellent.

### Embodiments for Carrying Out the Invention

The present invention will be described in detail below.

The active ingredient of the transdermal preparation of the present invention is compound A or a salt thereof. Compound A forms salts with various acidic compounds. Any one of them can be used in the present invention.

Examples of the salt of compound A include salts with mineral acids such as hydrochloric acid, hydrobromic acid, nitric acid, phosphoric acid and sulfuric acid; salts with carboxylic acids such as formic acid, acetic acid, citric acid, oxalic acid, fumaric acid, lactic acid, maleic acid, succinic acid, malic acid, tartaric acid, aspartic acid, trichloroacetic acid, trifluoroacetic acid, oleic acid and caprylic acid; and salts with sulfonic acids such as methanesulfonic acid, benzene sulfonic acid, p-toluene sulfonic acid, mesitylene sulfonic acid and naphthalene sulfonic acid. Salts with hydrochloric acid, lactic acid, methanesulfonic acid and acetic acid are preferable, and a salt with hydrochloric acid is more preferable. These include hydrates and solvates.

The concentration of the active ingredient of a transdermal preparation is, for example, 0.01 to 40 wt%, preferably 0.1 to 10 wt%, more preferably 0.3 to 6 wt%, and further preferably 1 to 3 wt%, based on the total amount of the preparation.

The transdermal absorption enhancers comprised in the transdermal preparation of the present invention include a higher alcohol, a higher monocarboxylic acid, a higher monocarboxylic acid ester, an aromatic monoterpene, a non-aromatic monoterpene having no polar group. One or more of the transdermal absorption enhancers may be comprised.

The higher alcohols include saturated alcohols having 8 to 18 carbon atoms such as octanol, nonanol, decanol, undecyl alcohol, lauryl alcohol, tridecyl alcohol, myristyl alcohol, pentadecyl alcohol, cetyl alcohol, heptadecyl alcohol and stearyl alcohol; and unsaturated alcohols having 8 to 18 carbon atoms such as oleyl alcohol, linoleyl alcohol and linolenyl alcohol. Octanol, decanol, lauryl alcohol, myristyl alcohol and oleyl alcohol are preferable, and oleyl alcohol is more preferable.

The higher monocarboxylic acids include saturated fatty acids having 8 to 18 carbon atoms such as caprylic acid, pelargonic acid, capric acid, undecylic acid, lauric acid, tridecylic acid, myristic acid, pentadecylic acid, palmitic acid, margaric acid and stearic acid; and unsaturated fatty acids having 8 to 18 carbon atoms such as citronellic acid, undecylenic acid, linderic acid, myristoleic acid, zoomaric acid, palmitoleic acid, oleic acid, linoleic acid and linolenic acid. Caprylic acid, capric acid, lauric acid, myristic acid, oleic acid, linoleic acid and linolenic acid are preferable, lauric acid, myristic acid and oleic acid are more preferable, and lauric acid and oleic acid are further preferable.

The higher monocarboxylic acid ester refers to a reaction product of a monocarboxylic acid having 6 to 18 carbon atoms and an alcohol having 1 to 6 carbon atoms. Examples thereof include ethyl caproate, isopropyl caproate, ethyl heptanoate, isopropyl heptanoate, ethyl caprylate, isopropyl caprylate, ethyl pelargonate, isopropyl pelargonate, ethyl caprate, isopropyl caprate, ethyl undecylate, isopropyl undecylate, ethyl laurate, isopropyl laurate, ethyl tridecylate, isopropyl tridecylate, ethyl myristate, isopropyl myristate, ethyl palmitate, isopropyl palmitate, ethyl oleate, isopropyl oleate, and the like. Ethyl caproate, ethyl laurate, isopropyl myristate and isopropyl palmitate are preferable, ethyl laurate, isopropyl myristate and isopropyl palmitate are more preferable, and isopropyl myristate is further preferable.

Examples of the aromatic monoterpene include cymene and thymol.

Examples of the non-aromatic monoterpene having no polar group include limonene, menthane, camphene, pinene, myrcene, ocimene, alloocimene, terpinolene, terpinene, phellandrene and carene. Limonene, menthane, camphene, pinene and alloocimene are preferable.

The transdermal preparation of the present invention is preferred to further comprise one or more non-aromatic monoterpenes having a polar group.

Examples of the non-aromatic monoterpene having a polar group include non-aromatic monoterpenes having a hydroxyl group, a carbonyl group or an ether bond.

Examples of the non-aromatic monoterpene having a polar group include geraniol, terpineol, menthol, borneol, isoborneol, nerol, camphor, citronellol, phenethyl alcohol, carveol, carvone, pulegone, piperitone, menthone, neomenthol, citronellal and cineole. Geraniol, menthol, borneol, camphor, menthone and cineole are preferable.

When the transdermal absorption enhancer comprised in the transdermal preparation of the present invention is one or more of a higher alcohol, a higher monocarboxylic acid, a higher monocarboxylic acid ester, an aromatic monoterpene and a non-aromatic monoterpene having no polar group, the concentration of each components of one or more transdermal absorption enhancers is 0.1 to 40 wt%, preferably 0.5 to 20 wt%, and more preferably 1 to 10 wt%, based on the total amount of the preparation.

Further, the concentration of the transdermal absorption enhancer can be reduced by comprising a non-aromatic monoterpene having a polar group. When a non-aromatic monoterpene having a polar group is comprised, the concentration of each component of one or more transdermal absorption enhancers selected from a higher alcohol, a higher monocarboxylic acid, a higher monocarboxylic acid ester, an aromatic monoterpene and a non-aromatic monoterpene having no polar group is, for example, 0.01 to 20 wt%, preferably 0.05 to 10 wt%, and more preferably 0.1 to 5 wt%, based on the total amount of the preparation. The concentration of the non-aromatic monoterpene having a polar group is, for example, 0.05 to 20 wt%, preferably 0.1 to 10 wt%, and more preferably 0.2 to 5 wt%, based on the total amount of the preparation.

Examples of the solvent used in the transdermal preparation of the present invention include a lower alcohol, a polyhydric alcohol and water. These may be used as a mixture.

Examples of the lower alcohol include ethanol, propanol and 2-propanol. Ethanol and 2-propanol are preferable.

Examples of the polyhydric alcohol include ethylene glycol, propylene glycol, 1,3-propanediol, 1,2-butanediol, 1,3-butanediol, 1,4-butanediol, 2,3-butanediol, 1,5-pentanediol, glycerin, dipropylene glycol, diethylene glycol, triethylene glycol, polyethylene glycol 400, and the like. Propylene glycol is preferable.

The transdermal preparation of the present invention can appropriately comprise a pH adjuster, a surfactant, a stabilizer, a preservative, an antioxidant, and the like, as needed.

The pH adjuster refers to an acid or a base normally used for adjusting pH in this field. Examples thereof include hydrochloric acid, phosphoric acid, sulfuric acid, methanesulfonic acid, citric acid, gluconic acid, succinic acid, lactic acid, acetic acid, sodium hydroxide, potassium hydroxide, sodium citrate, potassium carbonate, sodium hydrogen carbonate, sodium acetate, diisopropanolamine, diethanolamine, and the like.

The pH range of the transdermal preparation of the present invention is preferably 3 to 10.

The dosage form of the transdermal preparation of the present invention is not particularly limited as long as it can be applied to the skin. Examples thereof include a liquid form, a lotion form, an ointment form, a patch form, a cataplasm form, and the like. In the production of these dosage forms, raw materials normally used can be used. Examples thereof include white vaseline, purified lanolin, squalane, silicon, liquid paraffin, vegetable oil, wax, beeswax, water, a lower alcohol, a polyhydric alcohol and a water-soluble polymer.

As a base material of the patch form, a raw material for the base materials normally used can be used. Examples thereof include an acryl base material, a natural rubber base material, a silicon base material, a vinyl alcohol base material, a vinyl ester base material, a vinyl ether base material, and the like. Further, tackifiers such as rosin, rosin-modified maleic acid and a hydrogenated rosin ester may be used.

The transdermal preparation of the present invention can be produced by a common method in this field.

The liquid form or lotion form can be produced by using a solvent, an active ingredient and a transdermal absorption enhancer. Further, if necessary, other components may be used.

The ointment form can be produced by using a solvent, an active ingredient, a transdermal absorption enhancer and a thickening agent. Further, if necessary, other components may be used.

The patch form of a matrix diffusion control system is constituted of a backing layer, a drug storage layer, an adhesive layer and a separable protecting film. In the backing layer, drug storage layer, adhesive layer and separable protecting film constituting the preparation, base materials normally used can be widely used without any particular limitations. The patch form of a matrix diffusion control system can be produced by using a composition comprising an active ingredient and a transdermal absorption enhancer within the scope of the present invention, in the drug storage layer and/or the adhesive layer. Further, if necessary, other components may be used.

The patch form of a membrane permeation control system is constituted of a backing layer, a drug storage layer, a drug release control membrane, an adhesive layer and a separable protecting film. The patch form of a membrane transport control system can be produced by enclosing a composition comprising an active ingredient and a transdermal absorption enhancer within the scope of the present invention into the drug storage layer. Further, if necessary, other components may be used.

All components used in the present invention can be used by dissolving, suspending and/or emulsifying them by common methods.

The present invention will be described with Examples, Comparative Examples and Test Examples; however, the present invention is not limited by these.

If not otherwise specified, the "part(s)" means "part(s) by weight".

As an active ingredient, trihydrochloride salt pentahydrate of compound A was used.

### Example 1

The active ingredient (3 parts), oleyl alcohol (5 parts), propylene glycol (10 parts), ethanol (70 parts) and purified water (12 parts) were blended to obtain a preparation (100 parts).

### Example 2

The active ingredient (3 parts), oleyl alcohol (2 parts), propylene glycol (20 parts), 2-propanol (20 parts), ethanol (30 parts) and purified water (25 parts) were blended to obtain a preparation (100 parts).

### Example 3

The active ingredient (3 parts), caprylic acid (5 parts), propylene glycol (10 parts), ethanol (70 parts) and purified water (12 parts) were blended to obtain a preparation (100 parts).

### Example 4

The active ingredient (3 parts), lauric acid (5 parts), propylene glycol (10 parts), ethanol (70 parts) and purified water (12 parts) were blended to obtain a preparation (100 parts).

### Example 5

The active ingredient (3 parts), myristic acid (5 parts), propylene glycol (10 parts), ethanol (70 parts) and purified water (12 parts) were blended to obtain a preparation (100 parts).

### Example 6

The active ingredient (3 parts), oleic acid (5 parts), propylene glycol (10 parts), ethanol (70 parts) and purified water (12 parts) were blended to obtain a preparation (100 parts).

### Example 7

The active ingredient (3 parts), oleic acid (2 parts), propylene glycol (20 parts), 2-propanol (20 parts), ethanol (30 parts) and purified water (25 parts) were blended to obtain a preparation (100 parts).

### Example 8

The active ingredient (3 parts), ethyl caproate (5 parts), propylene glycol (10 parts), ethanol (70 parts) and purified water (12 parts) were blended to obtain a preparation (100 parts).

### Example 9

The active ingredient (3 parts), ethyl laurate (5 parts), propylene glycol (10 parts), ethanol (70 parts) and purified water (12 parts) were blended to obtain a preparation (100 parts).

### Example 10

The active ingredient (3 parts), isopropyl myristate (5 parts), propylene glycol (10 parts), ethanol (70 parts) and purified water (12 parts) were blended to obtain a preparation (100 parts).

### Example 11

The active ingredient (3 parts), isopropyl palmitate (5 parts), propylene glycol (10 parts), ethanol (70 parts) and purified water (12 parts) were blended to obtain a preparation (100 parts).

### Example 12

The active ingredient (3 parts), isopropyl myristate (10 parts), propylene glycol (10 parts), ethanol (70 parts) and purified water (7 parts) were blended to obtain a preparation (100 parts).

### Example 13

The active ingredient (3 parts), isopropyl myristate (1 part), propylene glycol (10 parts), ethanol (70 parts) and purified water (16 parts) were blended to obtain a preparation (100 parts).

### Example 14

The active ingredient (3 parts), isopropyl myristate (2 parts), propylene glycol (21 parts), 2-propanol (21 parts), ethanol (33 parts) and purified water (20 parts) were blended to obtain a preparation (100 parts).

### Example 15

The active ingredient (3 parts), (±)-limonene (5 parts), propylene glycol (10 parts), ethanol (69 parts) and purified water (13 parts) were blended to obtain a preparation (100 parts).

### Example 16

The active ingredient (3 parts), (±)-limonene (2 parts), propylene glycol (20 parts), 2-propanol (20 parts), ethanol (30 parts) and purified water (25 parts) were blended to obtain a preparation (100 parts).

### Example 17

The active ingredient (3 parts), eucalyptus oil (5 parts), propylene glycol (10 parts), ethanol (70 parts) and purified water (12 parts) were blended to obtain a preparation (100 parts).

### Example 18

The active ingredient (3 parts), oleyl alcohol (1 part), 1-menthol (1 part), propylene glycol (20 parts), 2-propanol (20 parts), ethanol (20 parts) and purified water (35 parts) were blended to obtain a preparation (100 parts).

### Example 19

The active ingredient (3 parts), oleic acid (1 part), 1-menthol (1 part), propylene glycol (15 parts), 2-propanol (20 parts), ethanol (20 parts) and purified water (40 parts) were blended to obtain a preparation (100 parts).

### Example 20

The active ingredient (3 parts), isopropyl myristate (3 parts), 1-menthol (5 parts), propylene glycol (10 parts), ethanol (70 parts) and purified water (9 parts) were blended to obtain a preparation (100 parts).

### Example 21

The active ingredient (3 parts), isopropyl myristate (0.5 parts), 1-menthol (2 parts), propylene glycol (20 parts), 2-propanol (20 parts), ethanol (30 parts) and purified water (24.5 parts) were blended to obtain a preparation (100 parts).

### Example 22

The active ingredient (3 parts), isopropyl myristate (0.5 parts), 1-menthol (2 parts), propylene glycol (20 parts), 2-propanol (20 parts), ethanol (30 parts), a 0.1 mol/L aqueous sodium hydroxide solution (9.1 parts) and purified water (15.4 parts) were blended to obtain a preparation (100 parts).

### Example 23

The active ingredient (3 parts), isopropyl myristate (0.6 parts), 1-menthol (0.5 parts), propylene glycol (20 parts), 2-propanol (20 parts), ethanol (30 parts) and purified water (25.9 parts) were blended to obtain a preparation (100 parts).

### Example 24

The active ingredient (3 parts), isopropyl myristate (1 part), geraniol (1 part), propylene glycol (20 parts), 2-propanol (20 parts), ethanol (30 parts) and purified water (25 parts) were blended to obtain a preparation (100 parts).

### Example 25

The active ingredient (3 parts), (±)-limonene (1 part), 1-menthol (1 part), propylene glycol (20 parts), 2-propanol (20 parts), ethanol (30 parts) and purified water (25 parts) were blended to obtain a preparation (100 parts).

### Example 26

The active ingredient (3 parts), (±)-limonene (1 part), geraniol (1 part), propylene glycol (20 parts), 2-propanol (20 parts), ethanol (30 parts) and purified water (25 parts) were blended to obtain a preparation (100 parts).

### Example 27

The active ingredient (3 parts), p-menthane (5 parts), propylene glycol (12 parts), ethanol (73 parts) and purified water (7 parts) were blended to obtain a preparation (100 parts).

### Example 28

The active ingredient (3 parts), p-menthane (2 parts), propylene glycol (21 parts), 2-propanol (21 parts), ethanol (33 parts) and purified water (20 parts) were blended to obtain a preparation (100 parts).

### Example 29

The active ingredient (3 parts), alloocimene (5 parts), propylene glycol (12 parts), ethanol (73 parts) and purified water (7 parts) were blended to obtain a preparation (100 parts).

### Example 30

The active ingredient (3 parts), 2-pinene (5 parts), propylene glycol (10 parts), ethanol (70 parts) and purified water (12 parts) were blended to obtain a preparation (100 parts).

### Example 31

The active ingredient (3 parts), 2-pinene (2 parts), propylene glycol (20 parts), 2-propanol (20 parts), ethanol (30 parts) and purified water (25 parts) were blended to obtain a preparation (100 parts).

### Example 32

The active ingredient (3 parts), camphene (5 parts), propylene glycol (10 parts), ethanol (70 parts) and purified water (12 parts) were blended to obtain a preparation (100 parts).

### Example 33

The active ingredient (3 parts), p-cymene (5 parts), propylene glycol (10 parts), ethanol (70 parts) and purified water (12 parts) were blended to obtain a preparation (100 parts).

### Example 34

The active ingredient (3 parts), thymol (5 parts), propylene glycol (10 parts), ethanol (70 parts) and purified water (12 parts) were blended to obtain a preparation (100 parts).

### Example 35

The active ingredient (3 parts), peppermint oil (5 parts), propylene glycol (10 parts), ethanol (70 parts) and purified water (12 parts) were blended to obtain a preparation (100 parts).

### Example 36

The active ingredient (3 parts), p-menthane (1 part), 1-menthol (1 part), propylene glycol (20 parts), 2-propanol (20 parts), ethanol (30 parts) and purified water (25 parts) were blended to obtain a preparation (100 parts).

### Example 37

The active ingredient (3 parts), 2-pinene (1 part), 1-menthol (1 part), propylene glycol (20 parts), 2-propanol (20 parts), ethanol (30 parts) and purified water (25 parts) were blended to obtain a preparation (100 parts).

### Example 38

The active ingredient (3 parts), (±)-limonene (1 part), borneol (1 part), propylene glycol (20 parts), 2-propanol (20 parts), ethanol (30 parts) and purified water (25 parts) were blended to obtain a preparation (100 parts).

### Example 39

The active ingredient (3 parts), (±)-limonene (1 part), 1,8-cineol (1 part), propylene glycol (20 parts), 2-propanol (20 parts), ethanol (30 parts) and purified water (25 parts) were blended to obtain a preparation (100 parts).

### Example 40

The active ingredient (3 parts), (±)-limonene (1 part), 1-menthone (1 part), propylene glycol (20 parts), 2-propanol (20 parts), ethanol (30 parts) and purified water (25 parts) were blended to obtain a preparation (100 parts).

### Example 41

The active ingredient (3 parts), (±)-limonene (1 part), (±)-camphor (1 part), propylene glycol (20 parts), 2-propanol (20 parts), ethanol (30 parts) and purified water (25 parts) were blended to obtain a preparation (100 parts).

### Example 42

The active ingredient (6 parts), isopropyl myristate (2 parts), 1-menthol (2 parts), propylene glycol (20 parts), 2-propanol (20 parts), ethanol (30 parts) and purified water (20 parts) were blended to obtain a preparation (100 parts).

### Example 43

The active ingredient (1 part), isopropyl myristate (0.5 parts), 1-menthol (2 parts), propylene glycol (20 parts), 2-propanol (20 parts), ethanol (30 parts) and purified water (26.5 parts) were blended to obtain a preparation (100 parts).

### Example 44

The active ingredient (0.3 parts), isopropyl myristate (0.5 parts), 1-menthol (2 parts), propylene glycol (20 parts), 2-propanol (20 parts), ethanol (30 parts) and purified water (27.2 parts) were blended to obtain a preparation (100 parts).

### Comparative Example 1

The active ingredient (3 parts), propylene glycol (10 parts), ethanol (70 parts) and purified water (17 parts) were blended to obtain a preparation (100 parts).

### Comparative Example 2

The active ingredient (3 parts), diisopropyl adipate (5 parts), propylene glycol (10 parts), ethanol (70 parts) and purified water (12 parts) were blended to obtain a preparation (100 parts).

### Comparative Example 3

The active ingredient (3 parts), diethyl sebacate (5 parts), propylene glycol (10 parts), ethanol (70 parts) and purified water (12 parts) were blended to obtain a preparation (100 parts).

### Comparative Example 4

The active ingredient (3 parts), diethyl phthalate (5 parts), propylene glycol (10 parts), ethanol (70 parts) and purified water (12 parts) were blended to obtain a preparation (100 parts).

### Comparative Example 5

The active ingredient (3 parts), salicylic acid (5 parts), propylene glycol (10 parts), ethanol (70 parts) and purified water (12 parts) were blended to obtain a preparation (100 parts).

### Comparative Example 6

The active ingredient (3 parts), polysorbate 80 (5 parts), propylene glycol (10 parts), ethanol (70 parts) and purified water (12 parts) were blended to obtain a preparation (100 parts).

### Comparative Example 7

The active ingredient (3 parts), urea (5 parts), propylene glycol (10 parts), ethanol (70 parts) and purified water (12 parts) were blended to obtain a preparation (100 parts).

### Comparative Example 8

The active ingredient (3 parts), 1-menthol (2 parts), propylene glycol (20 parts), 2-propanol (20 parts), ethanol (30 parts) and purified water (25 parts) were blended to obtain a preparation (100 parts).

### Comparative Example 9

The active ingredient (3 parts), geraniol (2 parts), propylene glycol (20 parts), 2-propanol (20 parts), ethanol (30 parts) and purified water (25 parts) were blended to obtain a preparation (100 parts).

### Comparative Example 10

The active ingredient (3 parts), borneol (2 parts), propylene glycol (20 parts), 2-propanol (20 parts), ethanol (30 parts) and purified water (25 parts) were blended to obtain a preparation (100 parts).

### Comparative Example 11

The active ingredient (3 parts), 1,8-cineole (2 parts), propylene glycol (20 parts), 2-propanol (20 parts), ethanol (30 parts) and purified water (25 parts) were blended to obtain a preparation (100 parts).

### Comparative Example 12

The active ingredient (3 parts), 1-menthone (2 parts), propylene glycol (20 parts), 2-propanol (20 parts), ethanol (30 parts) and purified water (25 parts) were blended to obtain a preparation (100 parts).

### Comparative Example 13

The active ingredient (3 parts), (±)-camphor (2 parts), propylene glycol (20 parts), 2-propanol (20 parts), ethanol (30 parts) and purified water (25 parts) were blended to obtain a preparation (100 parts).

### Test Example 1

An abdominal skin excised out from a male hairless rat (HWY/Slc, 8-weeks-old) was mounted in a Franz diffusion cell (effective diffusion area: 0.636 cm²) filled with phosphate buffered saline such that the dermal side faced the receptor side, and constant-temperature water at 32-33°C was circulated outside the cell. The preparation (0.5 mL) of each of Examples and Comparative Examples was applied to the stratum corneum side, and a receptor liquid (1.5 mL) was sampled every 3 hours (Microette, MODEL 57-12A-S manufactured by Hanson Research Corp.). The concentration of compound A in the receptor liquid sampled at each time point was measured by high performance liquid chromatography to obtain a 24 hours cumulative permeated amount.

### Measurement conditions

Detection device: UV ray photometer
Measurement wave length: 260 nm
Column: Symmetry Shield RP8 (manufactured by Waters Corp.), 3.5 µm, 4.6 × 100 mm
Precolumn: Symmetry Shield RP8 (manufactured by Waters Corp.), 3.5 µm, 3.9 × 20 mm
Column temperature: 40°C
Mobile phase: Methanesulfonic acid-triethylamine solution: water: methanol (5:70:25)

The methanesulfonic acid-triethylamine solution was prepared by the following method.

To water (300 mL), methanesulfonic acid (60 mL) and triethylamine (60 mL) were added and then water was added up to 600 mL of the total amount. To the solution, a 1 mol/L aqueous dipotassium hydrogen phosphate solution was added to adjust pH to 3.5. Flow rate: 0.6 mL/minute

The results of preparations of Examples 1, 3 to 6, 8 to 11, 15, 17, 27, 29, 30 and 32 to 35 and Comparative Examples 1 to 7 are shown in Table 1.

The preparations of Examples 1, 3 to 6, 8 to 11, 15, 17, 27, 29, 30 and 32 to 35 exhibited remarkably higher skin permeability than the preparation of Comparative Example 1, the preparations of Comparative Examples 2 and 3 comprising an aliphatic dicarboxylic acid diester, the preparation of Comparative Example 4 comprising an aromatic dicarboxylic acid diester, the preparation of Comparative Example 5 comprising an aromatic carboxylic acid, the preparation of Comparative Example 6 comprising a surfactant and the preparation of Comparative Example 7 comprising a softening agent.

**[Table 1]**

| | Transdermal absorption enhancer or additive | 24-hour Cumulative permeated amount (µg/cm²) |
|---|---|---|
| Example 1 | Oleyl alcohol | 3638 |
| Example 3 | Caprylic acid | 1228 |
| Example 4 | Lauric acid | 6587 |
| Example 5 | Myristic acid | 2895 |
| Example 6 | Oleic acid | 5860 |
| Example 8 | Ethyl caproate | 1346 |
| Example 9 | Ethyl laurate | 4106 |
| Example 10 | Isopropyl myristate | 3446 |
| Example 11 | Isopropyl palmitate | 3269 |
| Example 15 | (±)-Limonene | 2685 |
| Example 17 | Eucalyptus oil | 2819 |
| Example 27 | p-Menthane | 2201 |
| Example 29 | Alloocimene | 4363 |
| Example 30 | 2-Pinene | 2499 |
| Example 32 | Camphene | 3758 |
| Example 33 | p-Cymene | 4381 |
| Example 34 | Thymol | 6073 |
| Example 35 | Peppermint oil | 3297 |
| Comparative Example 1 | - | 29 |
| Comparative Example 2 | Diisopropyl adipate | 90 |
| Comparative Example 3 | Diethyl sebacate | 56 |
| Comparative Example 4 | Diethyl phthalate | 47 |
| Comparative Example 5 | Salicylic acid | 27 |
| Comparative Example 6 | Polysorbate 80 | 35 |
| Comparative Example 7 | Urea | 23 |

The results of preparations of Examples 12 and 13 are shown in Table 2.

The preparations of Examples 12 and 13 exhibited high skin permeability. Further, the more isopropyl myristate was added, the more the active ingredient was permeated.

**[Table 2]**

| | Weight of isopropyl myristate (%) | 24-hour Cumulative permeated amount (µg/cm²) |
|---|---|---|
| Example 12 | 10 | 3627 |
| Example 13 | 1 | 1241 |

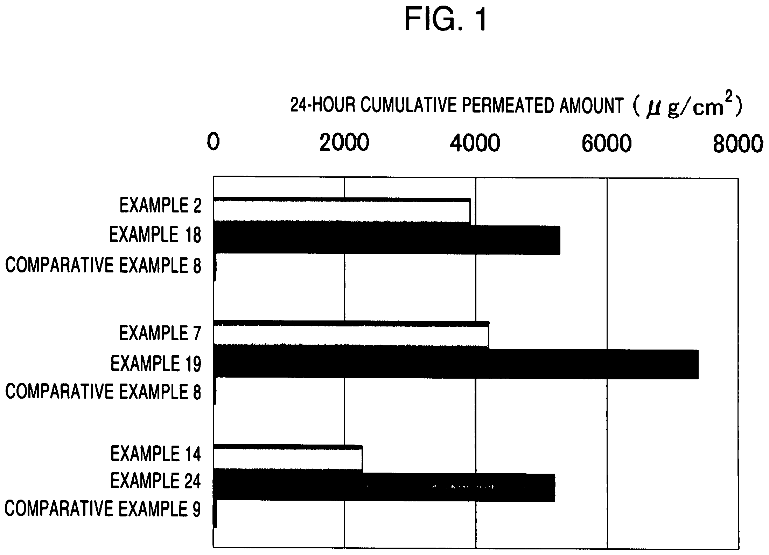

The results of preparations of Examples 2, 7, 14, 18, 19 and 24 and Comparative Examples 8 and 9 are shown in Table 3 and Fig. 1.

The preparations of Examples 2, 7 and 14 exhibited high skin permeability. The preparation of Example 18 exhibited higher skin permeability than the preparation of Comparative Example 8 and exhibited further higher skin permeability than the preparation of Example 2. The preparation of Example 19 exhibited higher skin permeability than the preparation of Comparative Example 8, and exhibited further higher skin permeability than the preparation of Example 7. The preparation of Example 24 exhibited higher skin permeability than the preparation of Comparative Example 9 and exhibited further higher skin permeability than the preparation of Example 14. The preparations of Examples 18, 19 and 24 exhibited high skin permeation enhancing effect by the combination use of a transdermal absorption enhancer with 1-menthol or geraniol.

**[Table 3]**

| | Example 2 | Example 7 | Example 14 | Example 18 | Example 19 | Example 24 | Comparative Example 8 | Comparative Example 9 |
|---|---|---|---|---|---|---|---|---|
| Active ingredient | 3 | 3 | 3 | 3 | 3 | 3 | 3 | 3 |
| Ethanol | 30 | 30 | 33 | 20 | 20 | 30 | 30 | 30 |
| 2-Propanol | 20 | 20 | 21 | 20 | 20 | 20 | 20 | 20 |
| Propylene glycol | 20 | 20 | 21 | 20 | 15 | 20 | 20 | 20 |
| Oleyl alcohol | 2 | - | - | 1 | - | - | - | - |
| Oleic acid | - | 2 | - | - | 1 | - | - | - |
| Isopropyl myristate | - | - | 2 | - | - | 1 | - | - |
| 1-Menthol | - | - | - | 1 | 1 | - | 2 | - |
| Geraniol | - | - | - | - | - | 1 | - | 2 |
| Purified water | 25 | 25 | 20 | 35 | 40 | 25 | 25 | 25 |
| 24-hour Cumulative permeated amount (µg/cm²) | 3923 | 4215 | 2273 | 5282 | 7396 | 5203 | 38 | 42 |

The results of preparations of Examples 20 and 23 are shown in Table 4.

The preparations of Examples 20 and 23 absorption exhibited high skin permeation enhancing effect by the combination use of a transdermal absorption enhancer with 1-menthol.

**[Table 4]**

| | Example 20 | Example 23 |
|---|---|---|
| Active ingredient | 3 | 3 |
| Ethanol | 70 | 30 |
| 2-Propanol | - | 20 |
| Propylene glycol | 10 | 20 |
| Isopropyl myristate | 3 | 0.6 |
| 1-Menthol | 5 | 0.5 |
| Purified water | 9 | 25.9 |
| 24-hour Cumulative permeated amount (µg/cm²) | 4929 | 2961 |

The results of preparations of Examples 21 and 22 are shown in Table 5.

The preparations of Examples 21 and 22 exhibited high skin permeability.

Additionally, the residual percent of active ingredient after storing the preparation of Example 21 at 40°C for 6 months was 99%. Thus, it was confirmed the preparation of Example 21 was a stable preparation.

**[Table 5]**

| | pH | 24-hour Cumulative permeated amount (µg/cm²) |
|---|---|---|
| Example 21 | 5.38 | 4662 |
| Example 22 | 7.62 | 6128 |

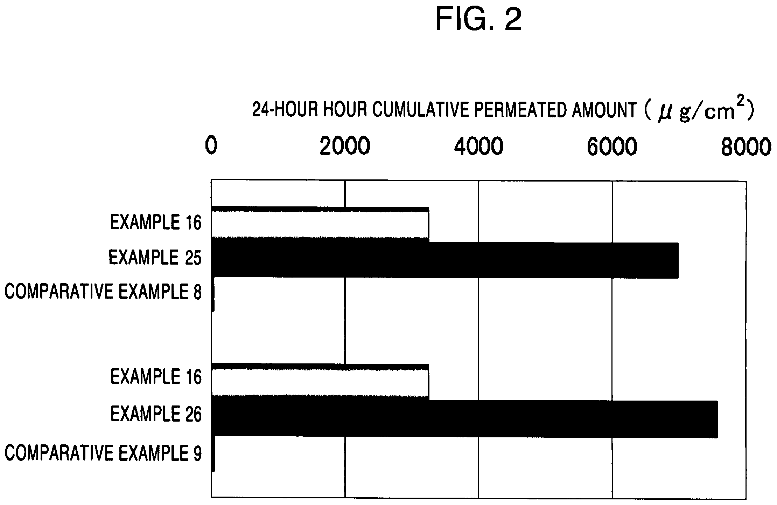

The results of preparations of Examples 16, 25, 26, 36 and 37 are shown in Table 6. Further, the results of preparations of Examples 16, 25 and 26 and preparations of Comparative Example 8 and 9 are shown in Fig. 2.

The preparation of Example 16 exhibited high skin permeability. The preparation of Example 25 exhibited higher skin permeability than the preparation of Comparative Example 8 and exhibited further higher skin permeability compared to the preparation of Example 16. Further, the preparation of Example 26 exhibited higher skin permeability than the preparation of Comparative Example 9, and exhibited further higher skin permeability compared to the preparation of Example 16. The preparations of Examples 25 and 26 exhibited high skin permeation enhancing effect by the combination use of a transdermal absorption enhancer with a non-aromatic monoterpene having a polar group.

The preparations of Examples 36 and 37 exhibited high skin permeation enhancing effect by the combination use of a transdermal absorption enhancer with a non-aromatic monoterpene having a polar group.

**[Table 6]**

| | Example 16 | Example 25 | Example 26 | Example 36 | Example 37 |
|---|---|---|---|---|---|
| Active ingredient | 3 | 3 | 3 | 3 | 3 |
| Ethanol | 30 | 30 | 30 | 30 | 30 |
| 2-Propanol | 20 | 20 | 20 | 20 | 20 |
| Propylene glycol | 20 | 20 | 20 | 20 | 20 |
| (±)-limonene | 2 | 1 | 1 | - | - |
| p-Menthane | - | - | - | 1 | - |
| 2-Pinene | - | - | - | - | 1 |
| 1-Menthol | - | 1 | - | 1 | 1 |
| Geraniol | - | - | 1 | - | - |
| Purified water | 25 | 25 | 25 | 25 | 25 |
| 24-hour Cumulative permeated amount (µg/cm²) | 3262 | 6994 | 7576 | 7220 | 6351 |

The results of preparations of Examples 38 to 41 and Comparative Examples 10 to 13 are shown in Table 7.

The preparations of Examples 38 to 41 exhibited high skin permeation enhancing effect by a combination use of a non-aromatic monoterpene having no polar group with a non-aromatic monoterpene having a polar group.

**[Table 7]**

| | Example 38 | Example 39 | Example 40 | Example 41 | Comparative Example 10 | Comparative Example 11 | Comparative Example 12 | Comparative Example 13 |
|---|---|---|---|---|---|---|---|---|
| Active ingredient | 3 | 3 | 3 | 3 | 3 | 3 | 3 | 3 |
| Ethanol | 30 | 30 | 30 | 30 | 30 | 30 | 30 | 30 |
| 2-Propanol | 20 | 20 | 20 | 20 | 20 | 20 | 20 | 20 |
| Propylene glycol | 20 | 20 | 20 | 20 | 20 | 20 | 20 | 20 |
| (±)-limonene | 1 | 1 | 1 | 1 | - | - | - | - |
| Borneol | 1 | - | - | - | 2 | - | - | - |
| 1,8-Cineol | - | 1 | - | - | - | 2 | - | - |
| 1-Menthone | - | - | 1 | - | - | - | 2 | - |
| (±)-Camphor | - | - | - | 1 | - | - | - | 2 |
| Purified water | 25 | 25 | 25 | 25 | 25 | 25 | 25 | 25 |
| 24-hour Cumulative permeated amount (µg/cm²) | 7375 | 5882 | 4899 | 4312 | 281 | 306 | 1036 | 118 |

### Brief Description of Drawings

[Fig. 1] Fig. 1 is a graph showing the effects of the combination use of a transdermal absorption enhancer with 1-menthol or geraniol.
[Fig. 2] Fig. 2 is a graph showing the effects of a combination use of (±)-limonene with 1- menthol or geraniol.

### Industrial Applicability

A transdermal preparation comprising 4-{3-[4-(3-{4-[amino(imino)methyl]phenoxy}propyl)-1-piperidinyl]propoxy}benzamidine or a salt thereof and a transdermal absorption enhancer has an excellent skin permeability, has a strong antifungal activity, improves patient's quality of life significantly, and is excellent in safety and useful for treating fungal infections.

## Claims

1. A transdermal preparation comprising 4-{3-[4-(3-{4-[amino(imino)methyl]phenoxy}propyl)-1-piperidinyl]propoxy}benzamidine or a salt thereof and a transdermal absorption enhancer, wherein the transdermal absorption enhancer is one or more transdermal absorption enhancer selected from a saturated alcohol having 8 to 18 carbon atoms, an unsaturated alcohol having 8 to 18 carbon atoms, a saturated fatty acid having 8 to 18 carbon atoms, an unsaturated fatty acid having 8 to 18 carbon atoms, a reaction product of a monocarboxylic acid having 6 to 18 carbon atoms and an alcohol having 1 to 6 carbon atoms, an aromatic monoterpene and a non-aromatic monoterpene having no polar group.

2. The transdermal preparation according to claim 1, wherein the saturated alcohol having 8 to 18 carbon atoms is octanol, nonanol, decanol, undecyl alcohol, lauryl alcohol, tridecyl alcohol, myristyl alcohol, pentadecyl alcohol, cetyl alcohol, heptadecyl alcohol or stearyl alcohol, the unsaturated alcohol having 8 to 18 carbon atoms is oleyl alcohol, linoleyl alcohol or linolenyl alcohol; the saturated fatty acid having 8 to 18 carbon atoms is caprylic acid, pelargonic acid, capric acid, undecylic acid, lauric acid, tridecylic acid, myristic acid, pentadecylic acid, palmitic acid, margaric acid or stearic acid, the unsaturated fatty acid having 8 to 18 carbon atoms is citronellic acid, undecylenic acid, linderic acid, myristoleic acid, zoomaric acid, palmitoleic acid, oleic acid, linoleic acid or linolenic acid; the reaction product of a monocarboxylic acid having 6 to 18 carbon atoms and an alcohol having 1 to 6 carbon atoms is ethyl caproate, isopropyl caproate, ethyl heptanoate, isopropyl heptanoate, ethyl caprylate, isopropyl caprylate, ethyl pelargonate, isopropyl pelargonate, ethyl caprate, isopropyl caprate, ethyl undecylate, isopropyl undecylate, ethyl laurate, isopropyl laurate, ethyl tridecylate, isopropyl tridecylate, ethyl myristate, isopropyl myristate, ethyl palmitate, isopropyl palmitate, ethyl oleate, or isopropyl oleate.

3. The transdermal preparation according to claim 1, wherein the saturated alcohol having 8 to 18 carbon atoms is octanol, decanol, lauryl alcohol, or myristyl alcohol, the unsaturated alcohol having 8 to 18 carbon atoms is oleyl alcohol; the saturated fatty acid having 8 to 18 carbon atoms is caprylic acid, capric acid, lauric acid, myristic acid, oleic acid, linoleic acid or linolenic acid; the reaction product of a monocarboxylic acid having 6 to 18 carbon atoms and an alcohol having 1 to 6 carbon atoms is ethyl caproate, ethyl laurate, isopropyl myristate or isopropyl palmitate; the aromatic monoterpene is cymene or thymol; and the non-aromatic monoterpene having no polar group is limonene, menthane, camphene, pinene or alloocimene.

4. The transdermal preparation according to any one of claims 1 to 3, further comprising one or more non-aromatic monoterpenes having a polar group.

5. The transdermal preparation according to claim 4, wherein the non-aromatic monoterpene having a polar group is geraniol, menthol, borneol, camphor, menthone or cineol.

6. The transdermal preparation according to any one of claims 1 to 3, wherein the concentration of each of components of the transdermal absorption enhancer is 0.1 to 40 wt% based on the total amount of the preparation.

7. The transdermal preparation according to claim 4 or 5, wherein the concentration of each of components of the transdermal absorption enhancer is 0.01 to 20 wt% based on the total amount of the preparation.

8. The transdermal preparation according to any one of claims 4 to 7, wherein the concentration of each of components of the non-aromatic monoterpene having a polar group is 0.05 to 20 wt% based on the total amount of the preparation.

## Patentansprüche

1. Transdermales Präparat, umfassend 4-{3-[4-(3-{4-[amino(imino)methyl]phenoxy}propyl)-1-piperidinyl]propoxy}benzamidin oder ein Salz desselben und einen Verstärker der transdermalen Absorption, wobei der Verstärker der transdermalen Absorption ein oder mehrere Verstärker der transdermalen Absorption ist, ausgewählt aus einem gesättigten Alkohol mit 8 bis 18 Kohlenstoffatomen, einem ungesättigten Alkohol mit 8 bis 18 Kohlenstoffatomen, einer gesättigten Fettsäure mit 8 bis 18 Kohlenstoffatomen, einer ungesättigten Fettsäure mit 8 bis 18 Kohlenstoffatomen, einem Reaktionsprodukt einer Monocarbonsäure mit 6 bis 18 Kohlenstoffatomen und einem Alkohol mit 1 bis 6 Kohlenstoffatomen, einem aromatischen Monoterpen und einem nicht-aromatischen Monoterpen, das keine polare Gruppe aufweist.

2. Transdermales Präparat nach Anspruch 1, wobei der gesättigte Alkohol mit 8 bis 18 Kohlenstoffatomen Octanol, Nonanol, Decanol, Undecylalkohol, Laurylalkohol, Tridecylalkohol, Myristylalkohol, Pentadecylalkohol, Cetylalkohol, Heptadecylalkohol oder Stearylalkohol ist, der ungesättigte Alkohol mit 8 bis 18 Kohlenstoffatomen Oleylalkohol, Linoleylalkohol oder Linolenylalkohol ist, die gesättigte Fettsäure mit 8 bis 18 Kohlenstoffatomen Caprylsäure, Pelargonsäure, Caprinsäure, Undecylsäure, Laurinsäure, Tridecylsäure, Myristinsäure, Pentadecylsäure, Palmitinsäure, Margarinsäure oder Stearinsäure ist, die ungesättigte Fettsäure mit 8 bis 18 Kohlenstoffatomen Citronellsäure, Undecylensäure, Linderinsäure, Myristoleinsäure, Zoomarinsäure, Palmitoleinsäure, Ölsäure, Linolsäure, oder Linolensäure ist, das Reaktionsprodukt einer Monocarbonsäure mit 6 bis 18 Kohlenstoffatomen und einem Alkohol mit 1 bis 6 Kohlenstoffatomen Ethylcaproat, Isopropylcaproat, Ethylheptanoat, Isopropylheptanoat, Ethylcaprylat, Isopropylcaprylat, Ethylpelargonat, Isopropylpelargonat, Ethylcaprat, Isopropylcaprat, Ethylundecylat, Isopropylundecylat, Ethyllaurat, Isopropyllaurat, Ethyltridecylat, Isopropyltridecylat, Ethyfmyristat, Isopropylmyristat, Ethylpalmitat, Isopropylpalmitat, Ethyloleat oder Isopropyloleat ist.

3. Transdermales Präparat nach Anspruch 1, wobei der gesättigte Alkohol mit 8 bis 18 Kohlenstoffatomen Octanol, Decanol, Laurylalkohol oder Myristylalkohol ist, der ungesättigte Alkohol mit 8 bis 18 Kohlenstoffatomen Oleylalkohol ist, die gesättigte Fettsäure mit 8 bis 18 Kohlenstoffatomen Caprylsäure, Caprinsäure, Laurinsäure, Myristinsäure, Ölsäure, Linolsäure oder Linolensäure ist, das Reaktionsprodukt einer Monocarbonsäure mit 6 bis 18 Kohlenstoffatomen und einem Alkohol mit 1 bis 6 Kohlenstoffatomen Ethylcaproat, Ethyllaurat, Isopropylmyristat oder Isopropylpalmitat ist, das aromatische Monoterpen Cymol oder Thymol ist und das nicht-aromatische Monoterpen, das keine polaren Gruppen aufweist, Limonen, Menthen, Camphen, Pinen oder Alloocimen ist.

4. Transdermales Präparat nach einem der Ansprüche 1 bis 3, ferner ein oder mehrere nicht-aromatische Monoterpene umfassend, die eine polare Gruppe aufweisen.

5. Transdermales Präparat nach Anspruch 4, wobei das nicht-aromatische Monoterpen, das eine polare Gruppe aufweist, Geraniol, Menthol, Borneol, Campher, Menton oder Cineol ist.

6. Transdermales Präparat nach einem der Ansprüche 1 bis 3, wobei die Konzentration jeder der Verbindungen des Verstärkers der transdermalen Absorption 0,1 bis 40 Gewichtsprozent, basierend auf der Gesamtmenge des Präparats, beträgt.

7. Transdermales Präparat nach Anspruch 4 oder 5, wobei die Konzentration jeder der Verbindungen des Verstärkers der transdermalen Absorption 0,01 bis 20 Gewichtsprozent, basierend auf der Gesamtmenge des Präparats, beträgt.

8. Transdermales Präparat nach einem der Ansprüche 4 bis 7, wobei die Konzentration jeder der Verbindungen des nicht-aromatischen Monoterpens, das eine polare Gruppe aufweist, 0,05 bis 20 Gewichtsprozent, basierend auf der Gesamtmenge des Präparats, beträgt.

## Revendications

1. Préparation à usage transdermique comprenant de la 4-{3-[4-(3-{4-[amino(imino)méthyl]phénoxy}propyl)-1-pipéridinyl]propoxy}benzamidine ou un sel de celle-ci et un amplificateur d'absorption par voie transdermique, dans laquelle l'amplificateur d'absorption par voie transdermique est un ou plusieurs amplificateur(s) d'absorption par voie transdermique choisi(s) parmi un alcool saturé possédant 8 à 18 atomes de carbone, un alcool insaturé possédant 8 à 18 atomes de carbone, un acide gras saturé possédant 8 à 18 atomes de carbone, un acide gras insaturé possédant 8 à 18 atomes de carbone, un produit de 1a réaction d'un acide monocarboxylique possédant 6 à 18 atomes de carbone et d'un alcool possédant 1 à 6 atomes de carbone, un monoterpène aromatique, et un monoterpène non aromatique ne possédant pas de groupe polaire.

2. Préparation à usage transdermique selon la revendication 1, dans laquelle l'alcool saturé possédant 8 à 18 atomes de carbone est l'octanol, le nonanol, le décanol, l'alcool undécylique, l'alcool laurylique, l'alcool tridécylique, l'alcool myristylique, l'alcool pentadécylique, l'alcool cétylique, l'alcool heptadécylique ou l'alcool stéarylique, l'alcool insaturé possédant 8 à 18 atomes de carbone est l'alcool oléylique, l'alcool linoléylique ou l'alcool linolénylique ; l'acide gras saturé possédant 8 à 18 atomes de carbone est l'acide caprylique, l'acide pélargonique, l'acide caprique, l'acide undécylique, l'acide laurique, l'acide tridécylique, l'acide myristique, l'acide pentadécylique, l'acide palmitique, l'acide margarique ou l'acide stéarique, l'acide gras insaturé possédant 8 à 18 atomes de carbone est l'acide citronellique, l'acide undécylénique, l'acide lindérique, l'acide myristoléique, l'acide zoomarique, l'acide palmitoléique, l'acide oléique, l'acide linoléique ou l'acide linolénique ; le produit de la réaction d'un acide monocarboxylique possédant 6 à 18 atomes de carbone et d'un alcool possédant 1 à 6 atomes de carbone est le caproate d'éthyle, le caproate d'isopropyle, l'heptanoate d'éthyle, l'heptanoate d'isopropyle, le caprylate d'éthyle, le caprylate d'isopropyle, le pélargonate d'éthyle, le pélargonate d'isopropyle, le caprate d'éthyle, le caprate d'isopropyle, l'undécylate d'éthyle, l'undécylate d'isopropyle, le laurate d'éthyle, le laurate d'isopropyle, le tridécylate d'éthyle, le tridécylate d'isopropyle, le myristate d'éthyle, le myristate d'isopropyle, le palmitate d'éthyle, le palmitate d'isopropyle, l'oléate d'éthyle, ou l'oléate d'isopropyle.

3. Préparation à usage transdermique selon la revendication 1, dans laquelle l'alcool saturé possédant 8 à 18 atomes de carbone est l'octanol, le décanol, l'alcool laurylique ou l'alcool myristylique, l'alcool insaturé possédant 8 à 18 atomes de carbone est l'alcool oléylique ; l'acide gras saturé possédant 8 à 18 atomes de carbone est l'acide caprylique, l'acide caprique, l'acide laurique, l'acide myristique, l'acide oléique, l'acide linoléique ou l'acide linolénique ; le produit de la réaction d'un acide monocarboxylique possédant 6 à 18 atomes de carbone et d'un alcool possédant 1 à 6 atomes de carbone est le caproate d'éthyle, le laurate d'éthyle, le myristate d'isopropyle ou le palmitate d'isopropyle ; le monoterpène aromatique est le cymène ou le thymol ; et le monoterpène non aromatique ne possédant pas de groupe polaire est le limonène, le menthane, le camphène, le pinène ou l'allo-ocimène.

4. Préparation à usage transdermique selon l'une quelconque des revendications 1 à 3, comprenant en outre un ou plusieurs monoterpène(s) non aromatique(s) possédant un groupe polaire.

5. Préparation à usage transdermique selon 1a revendication 4, dans laquelle le monoterpène non aromatique possédant un groupe polaire est le géraniol, le menthol, le bornéol, le camphre, la menthone ou le cinéol.

6. Préparation à usage transdermique selon l'une quelconque des revendications 1 à 3, dans laquelle la concentration de chacun des composants de l'amplificateur d'absorption par voie transdermique est de 0,1 à 40 % en poids par rapport à la quantité totale de la préparation.

7. Préparation à usage transdermique selon la revendication 4 ou 5, dans laquelle la concentration de chacun des composants de l'amplificateur d'absorption par voie transdermique est de 0,01 à 20 % en poids par rapport à la quantité totale de la préparation.

8. Préparation à usage transdermique selon l'une quelconque des revendications 4 à 7, dans laquelle la concentration de chacun des composants du monoterpène non aromatique possédant un groupe polaire est de 0,05 à 20 % en poids par rapport à la quantité totale de la préparation.
